(19) Europäisches Patentamt
European Patent Office
Office européen des brevets

(11) EP 2 552 385 B1

(12) # EUROPÄISCHE PATENTSCHRIFT

(45) Veröffentlichungstag und Bekanntmachung des Hinweises auf die Patenterteilung:
**26.07.2017 Patentblatt 2017/30**

(21) Anmeldenummer: **11708708.0**

(22) Anmeldetag: **28.02.2011**

(51) Int Cl.:
*A61K 8/25* (2006.01) *A61K 8/26* (2006.01)
*A61K 8/29* (2006.01) *A61K 8/35* (2006.01)
*A61K 8/55* (2006.01) *A61Q 19/04* (2006.01)

(86) Internationale Anmeldenummer:
**PCT/EP2011/000965**

(87) Internationale Veröffentlichungsnummer:
**WO 2011/116868 (29.09.2011 Gazette 2011/39)**

(54) **ZUBEREITUNGEN ENTHALTEND DIHYDROXYACETON**
PREPARATIONS COMPRISING DIHYDROXYACETONE
PRÉPARATIONS CONTENANT DE LA DIHYDROXYACÉTONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2010 EP 10003250**

(43) Veröffentlichungstag der Anmeldung:
**06.02.2013 Patentblatt 2013/06**

(73) Patentinhaber:
- **Merck Patent GmbH**
  **64293 Darmstadt (DE)**
- **Sachtleben Chemie GmbH**
  **47198 Duisburg (DE)**

(72) Erfinder:
- **BECK, Joern**
  **64342 Seeheim-Jugenheim (DE)**
- **GRAF, Ruediger**
  **64853 Otzberg (DE)**
- **PFLUECKER, Frank**
  **64297 Darmstadt (DE)**
- **JOHN, Stephan**
  **47198 Duisburg (DE)**
- **HIRTHE, Bernd**
  **47918 Toenisvorst (DE)**

(56) Entgegenhaltungen:
**EP-A1- 0 576 188** **WO-A1-2005/025531**
**WO-A2-92/17159** **DE-A1-102007 013 368**

- **DATABASE GNPD [Online] Mintel; Juli 2008 (2008-07), "Tanning Bed in a Tube", XP002670751, Database accession no. 943224**
- **DATABASE GNPD [Online] Mintel; September 2007 (2007-09), "Face Visage Self Tan for Face", XP002670752, Database accession no. 763035**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 552 385 B1

## Beschreibung

Die Erfindung betrifft Verfahren zur Herstellung spezieller Zubereitungen enthaltend Dihydroxyaceton neben mindestens einem Metalloxid und/oder - hydroxid, die sich dadurch auszeichnen, dass ein deutlich verringerter Abbau des Dihydroxyacetons nach Lagerung beobachtet wird.

Dihydroxyaceton (DHA) wird seit vielen Jahren als Selbstbräunungsmittel in der Kosmetik eingesetzt. In den letzten Jahren geht der Trend auch verstärkt dazu, Selbstbräunungsprodukte mit UV-Filtern, beispielsweise für die Tagespflege, zu kombinieren. Dies war in der Vergangenheit jedoch nur beschränkt möglich, da DHA in Kombination mit konventionellen anorganischen UV-Filtern, insbesondere $TiO_2$, einen starken Abbau während der Lagerung zeigt.

Die Aufgabe der Erfindung ist die Bereitstellung einer Methode zur Stabilisierung von Dihydroxyaceton in Zubereitungen, wenn die Zubereitungen neben Dihydroxyaceton mindestens ein Metalloxid und/oder -hydroxid enthalten.

Bisher wurde davon ausgegangen, dass die aktiven Zentren für die Wechselwirkung mit DHA an den anorganischen Metalloxiden oder Metallhydroxiden liegen und nur durch eine geeignete Oberflächengestaltung passiviert werden können.

In WO 92/17159 A2 wird die Verwendung von Alkylphosphaten zur Stabilisierung von Dihydroxyaceton zusammen mit einem Copolymeren in Öl-in-Wasser-Emulsionen beschrieben. Es wird jedoch kein Beispiel für eine Zubereitung beschrieben, die neben Dihydroxyaceton auch ein Metalloxid oder ein Metallhydroxid enthält.

In EP 576188 A1 wird eine Zubereitung enthaltend Pecosil PS-100 beschrieben.

Überraschenderweise wurde gefunden, dass durch Beimischung einer organischen Phosphorverbindung in eine Zubereitung enthaltend Dihydroxyaceton und mindestens ein Metalloxid und/oder -hydroxid, die Stabilität von Dihydroxyaceton in der kosmetischen Formulierung erhöht werden kann. Die Beimischung der organischen Phosphorverbindung kann jedoch nicht willkürlich erfolgen, sondern erfolgt nach den erfindungsgemäßen Verfahren, wie nachfolgend beschrieben.

Es ist von Vorteil, wenn die Beimischung der organischen Phosphorverbindung in eine Ölphase einer Zubereitung erfolgt, wenn die Ölphase darüber hinaus das Metalloxid und/oder Metallhydroxid enthält und die Zubereitung auch mindestens eine wässrige Phase enhält, die das Dihydroxyaceton enthält. Bei Herstellung der Zubereitung ist dabei die wässrige Phase frei von den organischen Phosphorverbindungen, die zur Stabilisierung verwendet werden. Es ist nicht auszuschließen, dass sich bei Lagerung ein Teil der organischen Phosphorverbindung an der Phasengrenze anreichern und/oder sich ein Teil in der wässrigen Phase löst. Es wurde jedoch gefunden, dass keine Stabilisierung zu beobachten ist, wenn bei Herstellung der Zubereitung die organische Phosphorverbindung der wässrigen Phase der Zubereitung beigemischt wird, die DHA enthält und sich das Metalloxid und/oder Metallhydroxid in der Ölphase der Zubereitung befindet.

Besonders bevorzugt ist die Zubereitung mit diesen Merkmalen ein Gel oder eine Öl-in-Wasser Emulsion.

Es wurde weiterhin gefunden, dass die Beimischung der organischen Phosphorverbindung in die wässrige Phase einer Zubereitung vorteilhaft ist, wenn die wässrige Phase neben der organischen

Phosphorverbindung auch Dihydroxyaceton und das Metalloxid und/oder Metallhydroxid enthält.

Beispiele für derartige Zubereitungen sind Hydrogele, die keine Ölphase enthalten. Beispiele für derartige Zubereitungen sind auch Gele, die einen geringen Anteil an Ölphase enthalten, beispielsweise Gele, die einen Gewichtsprozentanteil von 0,1 bis 5 Gewichtsprozent Ölanteil besitzen bezogen auf den Gesamtgehalt des Gels. Beispiele für derartige Zubereitungen sind jedoch auch Emulsionen, deren wässrige Phase die Komponenten organische Phosphorverbindung, Dihydroxyaceton und Metalloxid/Metallhydroxid enthalten. Bevorzugte Zubereitungen dieser Kategorie sind Gele oder Hydrogele. Ganz besonders bevorzugte Zubereitungen enthaltend mindestens eine wässrige Phase, die Dihydroxyaceton, ein Metalloxid und/oder Metallhydroxid und mindestens eine organische Phosphorverbindung enthalten und keine Ölphase enthalten sind Hydrogele.

Wird die organische Phosphorverbindung erfindungsgemäß in derartige Zubereitungen, wie zuvor beschrieben eingearbeitet, so verringert diese organische Phosphorverbindung den Abbau von Dihydroxyaceton bei Lagerung der Zubereitung im Vergleich zu Zubereitungen, die die gleichen Zubereitungsbestandteile enthalten ohne die organische Phosphorverbindung.

Je höher der Anteil der organischen Phosphorverbindung in den beschriebenen Zubereitungen ist, desto weniger DHA wird bei Lagerung abgebaut. Die Obergrenze des Anteils an organischer Phosphorverbindung wird bevorzugt dadurch bestimmt, dass die Formulierung ab einem gewissen Anteil an organischer Phosphorverbindung durch die organische Phosphorverbindung instabil wird, d.h. dass die Zubereitung beispielsweise inhomogen wird oder dass sich beispielsweise Wasser oder Öl abscheidet.

In einer bevorzugten Ausführungsform liegt der Anteil der organischen Phosphorverbindung in Gewichtsprozent im Bereich von 0,15 Gewichtsprozent bis 1,5 Gewichtsprozent bezogen auf die gesamte Zubereitung.
Alle Angaben zum Anteil an der mindestens einen organischen Phosphorverbindung sind demzufolge an die Bedingung geknüpft, dass die Zubereitung bei Anwesenheit dieser Menge an organischer Phosphorverbindung stabil bleibt.

Da die organische Phosphorverbindung zur Stabilisierung von Dihydroxyaceton bei Anwesenheit von Metalloxid und/oder Metallhydroxid in der Zubereitung verwendet wird, so ist es vorteilhaft, den Anteil an der zugesetzten organischen Phosphorverbindung auf den Anteil an Gesamtgehalt des Metalloxids und/oder Metallhydroxids zu beziehen. Der Gesamtgehalt bei zusätzlich beschichteten Metalloxiden oder Metallhydroxiden richtet sich hier immer auf die Gesamtpartikel.

In einer bevorzugten Ausführungsform liegt daher der Anteil der organischen Phosphorverbindung in Gewichtsprozent bezogen auf den Anteil des Metalloxids und/oder Metallhydroxids in der Zubereitung im Bereich von 3 Gewichtsprozent bis 30 Gewichtsprozent, bevorzugt bei 6 Gewichtsprozent bis 23 Gewichtsprozent, besonders bevorzugt bei 6 Gewichtsprozent bis 13 Gewichtsprozent.

Der Anteil an Dihydroxyaceton liegt bei 1 bis 12 Gewichtsprozent, bezogen auf die gesamte Zubereitung, vorzugsweise bei 1 bis 5 Gewichtsprozent.

Der Anteil an Metalloxid und/oder Metallhydroxid liegt bei 0.5 bis 25 Gewichtsprozent, bezogen auf die gesamte Zubereitung, vorzugsweise bei 1 bis 10 Gewichtsprozent, besonders bevorzugt bei 1 bis 5 Gewichtsprozent.

Im Sinne der vorliegenden Erfindung wird neben dem Begriff Zubereitung gleichbedeutend auch der Begriff Mittel oder Formulierung verwendet.

Alle Verbindungen oder Komponenten, die in den Zubereitungen verwendet werden können, sind entweder bekannt und käuflich erwerbbar oder können nach bekannten Verfahren synthetisiert werden.

Die organische Phosphorverbindung wird vorzugsweise ausgewählt aus der Gruppe der Hydroxyalkyldiphosphonsäuren oder deren Salze, Alkylphosphonsäuren, die durch mindestens eine COOH-Gruppe substituiert sein können oder deren Salze, Aminoalkylenphosphonsäuren oder deren Salze oder organische Phosphorsäureester oder deren Salze.

Hydroxyalkyldiphosphonsäuren sind beispielsweise Verbindungen der Formel I, wobei R eine lineare oder verzweigte Alkylgruppe mit 1 bis 20 C-Atomen bedeutet.

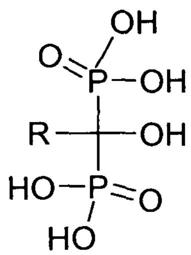

I

Die $C_1$-$C_{20}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl.

Bevorzugte Verbindungen der Formel I sind 1-Hydroxyethan-1,1-diphosphonsäure oder 1-Hydroxydodecan-1,1-diphosphonsäure. 1-Hydroxyethan-1,1-diphosphonsäure ist käuflich erwerbbar unter dem Namen Cublen® K 60 der Firma Zschimmer & Schwarz. 1-Hydroxydodecan-1,1-diphosphonsäure ist beispielsweise in dem Handelsprodukt Tensan AO der Firma Polygon Chemie enthalten (ca. 75%).

Bevorzugte Salze der Hydroxyalkyldiphosphonsäuren, sogenannte Hydroxyalkyldiphosphonate, sind Alkali- oder Erdalkalisalze der Verbindungen der Formel I, wie zuvor beschrieben, insbesondere Natrium- oder Kaliumsalze.

Alkylphosphonsäuren, die durch mindestens eine COOH-Gruppe substituiert sein können, sind beispielsweise Alkylphosphonsäuren mit einer linearen oder verzweigten Alkylgruppe mit 1 bis 20 C-Atomen, die gegebenenfalls mit mindestens einer COOH-Gruppe ersetzt sein können. Bevorzugte Alkylphosphonsäuren sind beispielsweise Laurylphosphonsäure oder Octylphosphonsäure, die von der Firma Rhodia kommerziell angeboten werden. Eine bevorzugte Alkylphosphonsäure mit mindestens einer COOH-Gruppe ist beispielweise 2-Phosphonobutan-1,2,4-tricarbonsäure, die von der Firma Zschimmer & Schwarz unter dem Markennamen Cublen® P 50 angeboten wird.

Bevorzugte Salze der Alkylphosphonsäuren, wie zuvor beschrieben, sind Alkali- oder Erdalkalisalze, insbesondere Natrium- oder Kaliumsalze.

Aminoalkylenphosphonsäuren sind beispielsweise Verbindungen der Formel II,

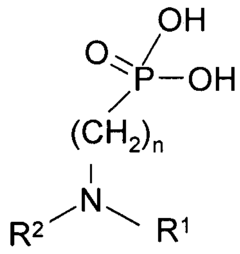

II

,

wobei n 1, 2, 3 oder 4 bedeutet, vorzugsweise 1 oder 2, und
$R^1$ und $R^2$ stehen jeweils unabhängig voneinander für $-(CH_2)_{n1}-P(O)(OH)_2$ oder $-(CH_2)n_1-N\{[(CH_2)_{n2}-P(O)(OH)_2][(CH_2)_{n3}-P(O)(OH)_2]\}$, wobei n1, n2 oder n3 jeweils unabhängig 1, 2, 3 oder 4 bedeuten, vorzugsweise 1 oder 2, bedeuten.
Bevorzugte Verbindungen der Formel II sind
Aminotrimethylenphosphonsäure,
Diethylentriaminpenta(methylenphosphonsäure) oder
Ethylendiamintetra(methylenphosphonsäure).
Aminotrimethylenphosphonsäure ist beispielsweise kommerziell erhältlich unter dem Markennamen Cublen® AP 5.

Bevorzugte Salze der Aminoalkylenphosphonsäuren sind Alkali- oder Erdalkalisalze der Verbindungen der Formel II, wie zuvor beschrieben, insbesondere Natrium- oder Kaliumsalze.

Organische Phosphorsäureester sind beispielsweise
Phosphorsäuremonoalkylester mit einer linearen oder verzweigten Alkylgruppe mit 1 bis 20 C-Atomen, Phosphorsäuredialkylester mit linearen oder verzweigten Alkylgruppen mit 1 bis 20 C-Atomen, die jeweils unabhängig voneinander ausgewählt werden, Phosphorsäuretrialkylester mit linearen oder verzweigten Alkylgruppen mit 1 bis 20 C-Atomen, die jeweils unabhängig voneinander ausgewählt werden, Salze der Phosphorsäuremonoalkylester oder Phosphorsäuredialkylester oder Gemische der genannten Phosphorsäureester, Phosphorsäure-Oxiester, Phosphorsäuremischester oder Nucleotide. Es können auch Gemische von Phosphorsäuremonoalkylestern und Phosphorsäuredialkylestern verwendet werden.

Phosphorsäuremonoalkylester sind beispielsweise
Phosphorsäuremonomethylester, Phosphorsäuremonoethylester, Phosphorsäuremonooctylester, Phosphorsäuremonocetylester, Phosphorsäuremonostearylester oder Phosphorsäuremonododecylester. Phosphorsäuremonodecylester ist beispielsweise kommerziell erhältlich unter dem Namen Hostaphat® CC100 der Firma Clariant oder Amphisol® A der Firma DSM. Ein Gemisch aus Phosphorsäure-mono- und distearylester ist kommerziell erhältlich unter dem Namen Hostaphat® CS120.

Die Alkylgruppen der Di- oder Trialkylester der Phosphorsäure können gleich oder verschieden sein. Bevorzugt sind sie gleich. Ausgewählte Di- oder Trialkylester sind beispielsweise Phosphorsäuredimethylester, Phosphorsäuretrimethylester, Phosphorsäurediethylester, Phosphorsäuretriethylester, Phosphorsäuredioctylester, Phosphorsäuretrioctylester, Phosphorsäuredicetylester, Phosphorsäuredistearylester oder Phosphorsäuredidodecylester.

Bevorzugte Salze der Phosphorsäuremonoalkylester oder Phosphorsäuredialkylester sind Alkali- oder Erdalkalisalze, insbesondere Natrium- oder Kaliumsalze. Kaliumcetylphosphat wird beispielsweise unter dem Handelsnamen Amphisol® K der Firma DSM angeboten.

Phosporsäure-Oxiester sind beispielsweise Phosphorsäureascorbylester oder Alkyltetraglycolether-Phosphorsäureester.

Ein Phosphorsäuremischester ist beispielsweise (3-Aminopropyl)-tocopheryl-phosphorsäureester.

Erfindungsgemäß einsetzbare Nucleotide sind beispielsweise Adenosin-5-Monophosphat (AMP), Adenosin-3,5-cyclophosphat (c-AMP), Adenosin-5-diphosphat (ADP) oder Adenosintriphosphat (ATP).

Besonders bevorzugt werden Phosphorsäuremonoalkylester oder deren Salze eingesetzt, wie zuvor beschrieben. Die Verwendung von Phosphorsäuremonocetylester ist insbesondere bevorzugt.

Das Metalloxid und/oder -hydroxid wird aus der Gruppe der Oxide bzw. Hydroxide von Silizium, Titan, Zink, Aluminium, Cer, Eisen, Yttrium oder Zirkonium oder Mischungen hieraus ausgewählt, die mit Metalloxiden bzw. -hydroxiden, organischen Säuren, ausgewählt aus der Gruppe Stearinsäure, Laurinsäure, Capronsäure oder Palmitinsäure, Polyolen, Polymeren oder silikonorganischen Verbindungen beschichtet sein können oder eine manganhaltige Beschichtung enthalten können.

Bevorzugt wird das Metalloxid und/oder -hydroxid ausgewählt aus Titandioxid oder Titanhydroxid oder Mischungen hieraus, die mit Metalloxiden oder -hydroxiden von Silizium, Zirkonium, Mangan und/oder Aluminium, mit Stearinsäure, Laurinsäure, Capronsäure oder Palmitinsäure, mit Glycerin, Sorbitol, Propylenglycol, Pentandiol, Hexandiol, Ethylhexyl-Glycerin, Pentaerythritol, Dipentaerythritol oder Trimethylolpropan oder mit Octyltrimethylsilan, Methicon,

Dimethicon (= Triethoxy-caprylsilan), Trimethoxycaprylsilan, Dimethicon/Methicon Copolymer, Diphenyl Capryl Methicone, Polymethyl-methacrylate dimethicone, Simethicon, Polyvinylpyrrolidon oder Polyethylen beschichtet sein können.

Besonders bevorzugt wird das Metalloxid und/oder -hydroxid ausgewählt aus Titandioxid oder Titanhydroxid oder Mischungen hieraus, die mit Metalloxiden oder -hydroxiden von Silizium und Mangan beschichtet sind, bevorzugt die mit Metalloxiden oder -hydroxiden von Silizium beschichtet sind, und die gegebenenfalls noch eine weitere Beschichtung aufweisen, wie beispielswiese eine Beschichtung mit einer organischen Säure, ausgewählt aus der Gruppe Stearinsäure, Laurinsäure, Capronsäure oder Palmitinsäure, Polyolen oder silikonorganischen Verbindungen. Polyole sind beispielsweise die Verbindungen Glycerin, Sorbitol, Propylenglycol, Pentandiol, Hexandiol, Ethylhexyl-Glycerin, Pentaerythritol, Dipentaerythritol oder Trimethylolpropan. Eine silikonorganische Verbindung ist beispielsweise Octyltrimethylsilan, Methicon, Dimethicon (= Triethoxy-caprylsilan), Trimethoxycaprylsilan, Dimethicon/Methicon Copolymer, Diphenyl Capryl Methicone, Polymethyl-methacrylate dimethicone oder Simethicon.

Der erfindungsgemäße Effekt der Stabilisierung von DHA kann bei Anwesenheit von Titandioxiden oder Titanhydroxiden oder Mischungen hieraus, die lediglich eine Beschichtung aus Metalloxiden oder -hydroxiden von Aluminium besitzen oder bei Einsatz von Aluminiumoxid/-hydroxid für das Metalloxid/-hydroxid je nach Wahl der organischen Phosphorverbindung geringer ausfallen. Falls derartige Metalloxide oder Metallhydroxide verwendet werden, so ist es zu empfehlen mindestens eine Beschichtung auf dieses Aluminiumoxid und/oder Aluminiumhydroxid aufzubringen, beispielsweise eine Beschichtung mit einer organischen Säure, ausgewählt aus der Gruppe Stearinsäure, Laurinsäure, Capronsäure oder Palmitinsäure, Polyolen, Polymeren oder silikonorganischen Verbindungen. Bei Verwendung von Phosphorsäuremonocetylester ist es zu empfehlen.

Geeignete Polymere sind beispielsweise Polyvinylpyrrolidon (PVP) oder Polyethylen (PE).

Bei den erfindungsgemäß herstellbaren Zubereitungen handelt es sich vorzugsweise um topisch anwendbare Zubereitungen, beispielsweise kosmetische oder dermatologische Formulierungen, besonders bevorzugt um kosmetische Formulierungen. Je nach gewünschtem Eigenschaftsprofil sind optional weitere geeignete Inhaltsstoffe enthalten. Topisch anwendbar bedeutet, dass die Zubereitung äußerlich und örtlich angewendet wird, d.h. dass die Zubereitung geeignet sein muss, um beispielsweise auf die Haut aufgetragen werden zu können. Bevorzugte Zubereitungen sind kosmetische Zubereitungen.

In einer weiteren Ausführungsform der vorliegenden Erfindung kann die erfindungsgemäß herstellbaree Zubereitung neben Dihydroxyaceton mindestens einen weiteren Selbstbräuner enthalten. Der Ausdruck Selbstbräuner wird synonym mit dem Ausdruck Selbstbräunungssubstanz verwendet.

Als vorteilhafte Selbstbräuner können unter anderem eingesetzt werden:

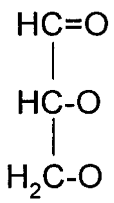

Glycerolaldehyd

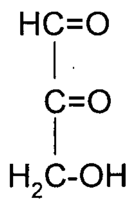

Hydroxymethylglyoxal

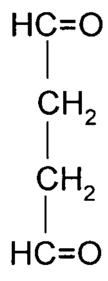

γ-Dialdehyd

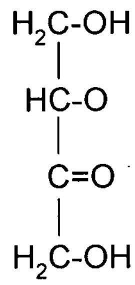

Erythrulose

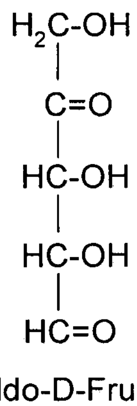

6-Aldo-D-Fructose

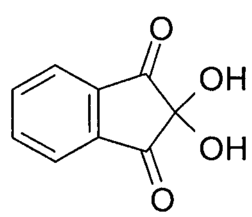

Ninhydrin

Ferner ist das 5-Hydroxy-1,4-naphtochinon (Juglon) zu nennen, das aus den Schalen frischer Walnüsse extrahiert wird sowie das in den Henna-Blättern vorkommende 2-Hydroxy-1,4-naphtochinon (Lawson).

Die erfindungsgemäß herstellbaren Zubereitungen, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Auch bei kombinierter Verwendung mit weiteren Selbstbräunern, wie zuvor beschrieben, ist die Entstehung von Fehlgerüchen nicht auszuschließen. Daher kann die erfindungsgemäße Zubereitung vorzugsweise auch Formaldehydfänger sowie gegebenenfalls Flavonoide zur Verbesserung des Geruches enthalten.

Vorzugsweise wird der Formaldehydfänger ausgewählt aus der Gruppe Alkalimetall-, Erdalkali-metall- oder Ammoniumbisulfit. Besonders bevorzugt ist eine Zubereitung, die in Kombination DHA Plus, eine Mischung aus DHA, Natriumbisulfit und Magnesiumstearat, enthält.

DHA Plus ist eine Produktmischung, welche zur Maskierung, Eliminierung oder Neutralisierung des Formaldehyds Natriumbisulfit, gleichbedeutend mit $Na_2S_2O_5$ oder INCI: sodium disulfite, enthält. Der Zusatz von Natriumbisulfit in fertigen Formulierungen führt zur signifikanten Reduktion oder Unterbindung des unangenehmen Geruchs. DHA Plus wird von der Firma Merck, Darmstadt vertrieben.

Das gegebenenfalls in der erfindungsgemäß herstellbaren Zubereitung vorhandene Flavonoid wirkt dabei zusätzlich als Stabilisator für den Selbstbräuner bzw. die Selbstsbräunungssubstanzen und/oder reduziert oder vermeidet oder verbessert lagerabhängige Fehlgerüche, die auch durch enthaltene Zusatz- oder Hilfsstoffe entstehen können.

Vorzugsweise handelt es sich um ein Flavonoid, bei dem eine oder mehrere phenolische Hydroxygruppen durch Veretherung oder Veresterung blockiert sind. Beispielsweise haben sich Hydroxyethyl-substituierte Flavonoide, wie vorzugsweise Troxerutin, Troxequercetin, Troxeisoquercetin oder Troxeluteolin, und Flavonoid-sulfate oder Flavonoid-phosphate, wie vorzugsweise Rutinsulfate, dabei als besonderes gut geeignete Flavonoide erwiesen. Besonders bevorzugte Flavonoide sind Rutinsulfat und Troxerutin. Ganz besonders bevorzugt ist die Verwendung von Troxerutin.

Die bevorzugten Flavonoide verfügen über einen nicht positiv geladenen Flavangrundkörper. Es wird vermutet, dass durch diese Flavonoide Metallionen wie z.B. $Fe^{2+}/Cu^{2+}$ komplexiert werden und so Autooxidations-vorgänge bei Duftstoffen oder Verbindungen, deren Abbau zu Fehlgerüchen führt, verhindert bzw. vermindert werden.

Besonders bevorzugt ist eine Zubereitung, wie zuvor beschrieben, die DHA Rapid und/oder Natriumbisulfit enthält. DHA Rapid ist eine Produktmischung enthaltend Dihydroxyaceton und Troxerutin, der Firma Merck, Darmstadt.

Entsprechende Vormischungen und Zubereitungen, die Formaldehydfänger sowie gegebenenfalls Flavonoide zur Verbesserung des Geruches auf der Haut enthalten, sind in der Deutschen Patentanmeldung mit dem Anmeldeaktenzeichen DE 10 2007 013 368.7 beschrieben.

Des Weiteren können die folgenden Substanzen alleine oder in Kombination mit DHA zur Bräunungsintensivierung in den erfindungsgemäß herstellbaren Zubereitungen eingesetzt werden:

- Vegetan Premium (INCI Dihydroxyacetone / Melanin) von Soliance,
- MelanoBronze (INCI: Vitex agnus castus extract (and) acetyl tyrosine (and) glycerin (and) alcohol (and) water (aqua)) Mibelle AG Biochemistry.
- Instabronze® (INCI Dihydroxyaceton (DHA), N-acétyl tyrosine), Alban-Müller,
- Substanzen welche penetrationsfördernd wirken, wie z.B. Leerliposome, Propylenglykol, Isohexadecan (Arlamol HD; Croda), Dimethylisosorbid (Arlasolve DMI; CRODA), Trimethylpentanediol/Adipic Acid/Isononanoic Acid Copolymer (INCI-Bezeichnung) oder (Lexorez TC-8, Inolex).

Bevorzugte Zubereitungen enthalten dabei mindestens einen organischen UV-Filter, insbesondere ein Dibenzoylmethanderivat. Die im Rahmen der vorliegenden Erfindung verwendeten Dibenzoylmethanderivate sind an sich bereits wohlbekannte Produkte, die insbesondere in den oben genannten Druckschriften FR-A-2 326 405, FR-A-2 440 933 und EP-A-0 114 607 beschrieben sind.

Die Dibenzoylmethanderivate können insbesondere unter den Dibenzoylmethanderivaten der folgenden Formel ausgewählt sein:

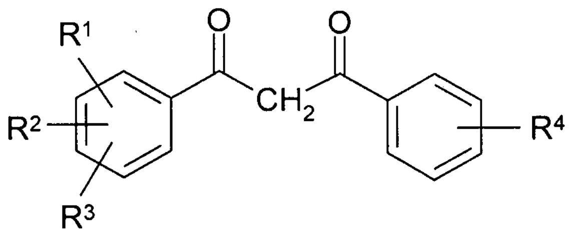

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Es können selbstverständlich ein Dibenzoylmethanderivat oder mehrere Dibenzoylmethanderivate verwendet werden. Von den Dibenzoylmethanderivaten können insbesondere:

2-Methyldibenzoylmethan, 4-Methyldibenzoylmethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4,4'-Methoxy-tert.-butyldibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan und 2,6-Dimethyl-4-tert.-butyl-4'-methoxydibenzoylmethan genannt werden, wobei diese Aufzählung nicht einschränkend ist. Von den obengenannten Dibenzoylmethanderivaten wird insbesondere das 4,4'-Methoxy-tert.-butyldibenzoylmethan und insbesondere das unter der Handelsbezeichnung Eusolex® 9020 von der Firma Merck im Handel befindliche 4,4'-Methoxy-tert.-butyldibenzoylmethan bevorzugt, wobei dieser Filter der folgenden Strukturformel entspricht:

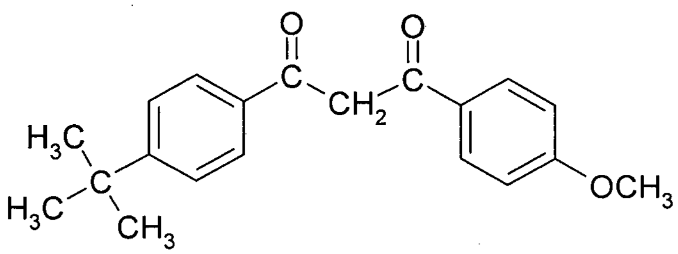

Ein weiteres bevorzugtes Dibenzoylmethanderivat ist das 4-Isopropyldibenzoylmethan.

Weitere bevorzugte Zubereitungen mit alternativen organischen UV-Filtern enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenonderivat(e) können in den erfindungsgemäß herstellbaren Zubereitungen in Mengenanteilen vorliegen, die im Allgemeinen im Bereich von 0,1 Gew.-% bis 10 Gew.-% (Gewichtsprozent) liegen und vorzugsweise in Mengenanteilen, die im Bereich von 0,3 Gew.-% bis 5 Gew.-% liegen, wobei diese Mengenanteile auf das Gesamtgewicht der Zubereitung bezogen sind.

Die erfindungsgemäß herstellbaren Zubereitungen können selbstverständlich einen oder mehrere zusätzliche(n) hydrophile(n) oder lipophile(n) Sonnenschutzfilter, die im UV-A-Bereich und/oder UV-B-Bereich und(/oder IR und/oder VIS-Bereich (Absorber) wirksam sind, enthalten. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere zu nennen sind hier:

- para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Haarmann and Reimer, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Haarmann and Reimer.

β,β-Diphenylacrylate Derivate: Octocrylene, z. B. vetrieben unter dem Namen "Eusolex OCR" von der Fa. Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate.

Benzylidencampher Derivate: 3-Benzylidenecamphor, z. B. vetrieben unter dem Namen "Mexoryl SD" von der

Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Haarmann and Reimer.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazine Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine.

Anthranilin Derivate: Menthyl anthranilate, z. B. vetrieben unter dem Namen "Neo Heliopan MA" von der Fa. Haarmann and Reimer.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung

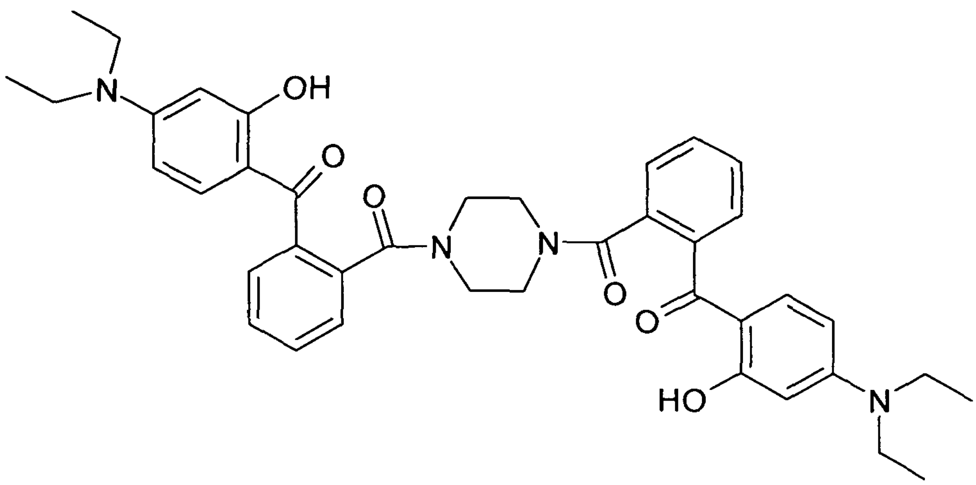

Die in der Liste aufgeführten Verbindungen sind nur als Beispiele aufzufassen. Selbstverständlich können auch andere UV-Filter verwendet werden.

Die zum Einsatz geeigneten organischen UV-schützende Substanzen sind bevorzugt aus der folgenden Liste auszuwählen: Ethylhexyl salicylate, Octocrylene, Butylmethoxydibenzoylmethane, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in die Formulierungen eingearbeitet.

Organische UV-Filter werden insgesamt in der Regel in einer Menge von 0,01 Gewichtsprozent bis 20 Gewichtsprozent, vorzugsweise 0,5 Gew.-% bis 20 Gew.-%, in die Formulierungen eingearbeitet.

Alle genannten UV-Filter können auch in verkapselter Form eingesetzt werden. Vorteilhaft ist es dabei, wenn die Kapseln so klein sind, dass sie mit dem bloßen Auge nicht beobachtet werden können.

Geeignete Kapseln können Wände aus anorganischen oder organischen Polymeren aufweisen. Beispielsweise

wird in US 6,242,099 B1 die Herstellung geeigneter Kapseln mit Wänden aus Chitin, Chitin-Derivaten oder polyhydroxylierten Polyaminen beschrieben. Besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxidhydroxid) aufgebaut sind. Die Herstellung entsprechender Kapseln ist dem Fachmann beispielsweise aus den zitierten Patentanmeldungen bekannt.

Dabei sind die Kapseln in erfindungsgemäß herstellbaren Zubereitungen vorzugsweise in solchen Mengen enthalten, die gewährleisten, dass die verkapselten UV-Filter in den oben angegebenen Mengen in der Zubereitung vorliegen.

Ferner können die erfindungsgemäß herstellbaren Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer,

Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

Es kann ferner günstig sein, als Farbstoff eine oder mehrere Substanzen aus der folgenden Gruppe zu wählen:

> 2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, ß-Carotin oder Cochenille.

Vorteilhaft sind ferner Zubereitungen mit einem Gehalt an Perlglanzpigmenten oder Interferenzpigmenten, insbesondere solchen Pigmenten, deren Verträglichkeit mit Dihydroxyaceton schon festgestellt wurde. Bevorzugt sind insbesondere die Perlglanzpigmente bzw. Interferenzpigmente, die auf einem plättchenförmigen Substrat basieren und mit Titandioxid und/oder Eisenoxid ($Fe_2O_3$) beschichtet sind und gegebenenfalls eine finale $SiO_2$-Schicht aufweisen.

Besonders bevorzugte Pigmente sind z. B. die von der Firma Merck unter den Handelsnamen Timiron®, Colorona®, Dichrona® oder Sirona® erhältlichen Pigmente.

Die Gesamtmenge der Farbstoffe und farbgebenden Pigmente wird vorteilhaft aus dem Bereich von z. B. 0,1 Gew.-% bis 30 Gew.-%, vorzugsweise von 0,5 Gew.-% bis 15 Gew.-%, insbesondere von 1,0 Gew.-% bis 10 Gew.-% gewählt, jeweils bezogen auf das Gesamtgewicht der Zubereitungen.

Die schützende Wirkung gegen oxidativen Stress bzw. gegen die Einwirkung von Radikalen kann weiter verbessert werden, wenn die Zubereitungen ein oder mehrere Antioxidantien enthalten.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, Pentanatriumethylendiamintetramethylenphosphonat (INCI: Pentasodium ethylenediamine tetramethylene phosphonate), ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B.

Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B

A 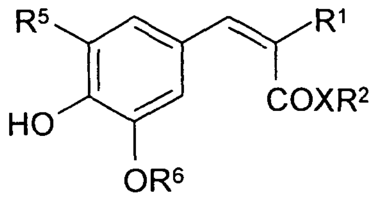 oder 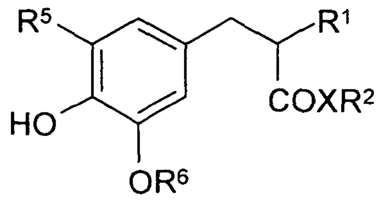 B ,

worin

$R^1$ aus der Gruppe $-C(O)CH_3$, $-CO_2R^3$, $-C(O)NH_2$ und $-C(O)N(R^4)_2$ ausgewählt werden kann,
X O oder NH,
$R^2$ lineares oder verzweigtes Alkyl mit 1 bis 30 C-Atomen,
$R^3$ lineares oder verzweigtes Alkyl mit 1 bis 20 C-Atomen,
$R^4$ jeweils ungabhängig voneinander H oder lineares oder verzweigtes Alkyl mit 1 bis 8 C-Atomen,
$R^5$H oder lineares oder verzweigtes Alkyl mit 1 bis 8 C-Atomen oder lineares oder verzweigtes Alkoxy mit 1 bis 8 C-Atomen und $R^6$ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzy)-malonsäure-bis-(2-ethylhexyl)ester (z.B. RonaCare® AP). Die genannten Verbindungen der Formel A oder B sind auch vorteilhafte Photostabilisatoren für organische UV-Filter, aber auch anderen photosensitiven Verbindungen, wie beispielsweise Parfum oder Farbstoffe.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die erfindungsgemäß herstellbaren Zubereitungen können als weitere Inhaltsstoffe Vitamine enthalten. Bevorzugt sind Vitamine und Vitamin-Derivate aus gewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäß herstellbaren Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Dabei wird vorzugsweise eine Pyrimidincarbonsäure gemäß der unten stehenden Formel II eingesetzt,

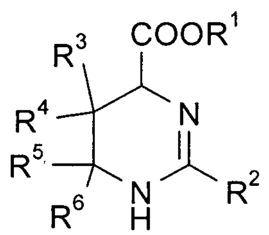

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die Zubereitungen derartige Pyrimidincarbonsäuren vorzugsweise in Mengen bis zu 15 Gew.-%.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0,01 Gew.-% bis 10 Gew.-% des Aryloxims, wobei es insbesondere bevorzugt ist, wenn die Zubereitung 0,05 Gew.-% bis 5 Gew-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Die erfindungsgemäß herstellbaren Zubereitungen mit den Merkmalen, wie zuvor beschrieben, können insbesondere in Form von Cremes, Milchen, Gelen oder Gel-Cremes vorliegen und gegebenenfalls können sie als Aerosole konfektioniert sein und in Form von Schäumen oder Sprays vorliegen.

Ein Gegenstand der Erfindung ist das Verfahren zur Herstellung einer Zubereitung in Form einer Öl-in-Wasser-Emulsion, dadurch gekennzeichnet, dass der Ölphase der Zubereitung die organische Phosphorverbindung und anschließend das Metalloxid und/oder Metallhydroxid beigemischt wird und bei maximal 40°C die mindestens eine wässrige Phase enthaltend Dihydroxyaceton zugegeben und homogenisiert wird, wobei die organische Phosphorverbindung und das Metalloxid und/oder Metallhydroxid entsprechend Anspruch 1 gewählt wird.

Ein weitere Gegenstand der Erfindung ist ein Verfahren zur Herstellung einer Zubereitung in Form eines Hydrogels nach Anspruch 2, dadurch gekennzeichnet, dass eine wässrige Lösung enthaltend die mindestens eine organische Phosphorverbindung und das Metalloxid und/oder Metallhydroxid vorgelegt wird und eine Mischung aus einem Gelbildner und Dihydroxyaceton in Wasser zugegeben wird und homogenisiert wird.

Die erfindungsgemäß herstellbaren kosmetischen Zubereitungen können als Zubereitung zum Schutz der menschlichen Epidermis oder der Haare gegen UV-Strahlung, zur Hautpflege, als Sonnenschutzmittel , Selbstbräunungsmittel oder Schminkprodukte verwendet werden.

Es soll darauf hingewiesen werden, dass in den Formulierungen zum Sonnenschutz, die einen Träger vom Typ einer Öl-in-Wasser-Emulsion aufweisen, die wässrige Phase im Allgemeinen 50 Gew.-% bis 95 Gew.-% und vorzugsweise 70 Gew.-% bis 90 Gew.-%, bezogen auf die gesamte Formulierung, die Ölphase 5 Gew.-% bis 50 Gew.-% und vorzugsweise 10 Gew.-% bis 30 Gew.-%, bezogen auf die gesamte Formulierung und der (Co)emulgator oder die (Co)emulgatoren 0,5 Gew.-% bis 20 Gew.-% und vorzugsweise 2 Gew.-% bis 10 Gew.-%, bezogen auf die gesamte Formulierung, ausmachen.

Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Erfindungsgemäß herstellbare Emulsionen sind vorteilhaft und enthalten z. B. -die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen

niedriger C-Zahl, z.B. mit Isopropanol, Propylenglykol oder Glycerin, oder Ester von Fett-lkoholen mit Alkansäuren niedriger C-Zahl oder mit Fettsäuren;

- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäß herstellbaren Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet.

In einer bevorzugten Ausführungsform enthalten die erfindungsgemäß herstellbaren Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Die erfindungsgemäß herstellbaren Zubereitungen sind vorteilhaft dadurch gekennzeichnet, dass das hydrophile Tensid oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zubereitung, vorliegt oder vorliegen.

Als Emulgatoren können beispielsweise die bekannten O/W-Emulgatoren verwendet werden. Es ist vorteilhaft, weitere übliche Co-emulgatoren in den erfindungsgemäß bevorzugten O/W-Emulsionen zu verwenden.

Als erfindungsgemäß besonders bevorzugter Emulgator für O/W-Emulsionen hat sich das Handelsprodukt Ceralution C der Firma Sasol erwiesen.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)-stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)-isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol-(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,
Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Die Zubereitungen, wie zuvor beschrieben, können die genannten notwendigen oder optionalen Bestandteile/Inhaltsstoffe enthalten oder umfassen, daraus im Wesentlichen oder daraus bestehen.

Im folgenden wird die Erfindung anhand von Beispielen näher erläutert.

**Beispiel:**

Experimentelle Untersuchungen zum DHA-Abbau und/oder Verfärbungen der Zubereitungen enthaltend DHA und Metalloxid und/oder Metallhydroxid, wie in den Tabellen angezeigt:

Beispiel 1A): In den Untersuchungen wurde die nachfolgend beschriebene O/W-Testformulierung verwendet:

| **Handelsname** | **INCI** | |
|---|---|---|
| | | **%** |
| **A** | | |
| **$TiO_2$** | | ***5,00*** |
| Tego Care 150 | GLYCERYL STEARATE, STEARETH-25, CETETH-20; STEARYL ALCOHOL | *8,00* |
| Lanette O | *CETEARYL ALCOHOL* | *1,50* |
| Tegosoft liquid | *CETEARYL ETHYLHEXANOATE* | *5,00* |
| Miglyol 812N | *CAPRYC/CAPRYLIC TRIGLYVCERIDE* | *5,00* |
| Abil-Wax 2434 | *STEAROXY DIMETHICONE* | *1,00* |
| DC 200 (100cs) | *DIMETHICONE* | *0,50* |
| Propylparaben | *PROPYLPARABEN* | *0,05* |
| Phosphorsäuremonocetylester | *Cetyl Phosphate* | *0,15bis 1,5* |
| **B** | | |
| 1,2-Propandiol | *PROPYLEN GLYCOL* | *3,00* |
| Methylparaben | *METHYL PARABEN* | *0,15* |
| Wasser | *AQUA* | *Ad 100* |
| **C** | | |
| **DHA** | ***DIHYDROXYACETONE*** | ***5,00*** |
| Wasser | *AQUA* | *10,00* |
| | | *100,00* |

Herstellung:

**Phase A** ohne $TiO_2$ und Phase **B** werden getrennt auf 80°C erhitzt. $TiO_2$ unter Rühren in **Phase A** geben. **Phase B** langsam in **Phase A** einrühren. Homogenisieren (1 Minute mit ESGE Zauberstab Stufe II). Unter Rühren abkühlen und bei 40°C **Phase C** zugeben.

Für den Lagerversuch wird die oben genannte Testformulierung mit 5 Gewichtsprozent DHA zusammen mit unterschiedlichen Titandioxid-Qualitäten untersucht. Sollte sich ein Lagerversuch auf die Testformulierung enthaltend 2 Gewichtsprozent DHA beziehen, so ist dies entsprechend angegeben.

Der DHA-Abbau in den untersuchten Testformulierungen wird wie folgt festgestellt:

DHA wird durch eine enzymatische Bestimmungsmethode analysiert und basiert auf der folgenden Reaktion:

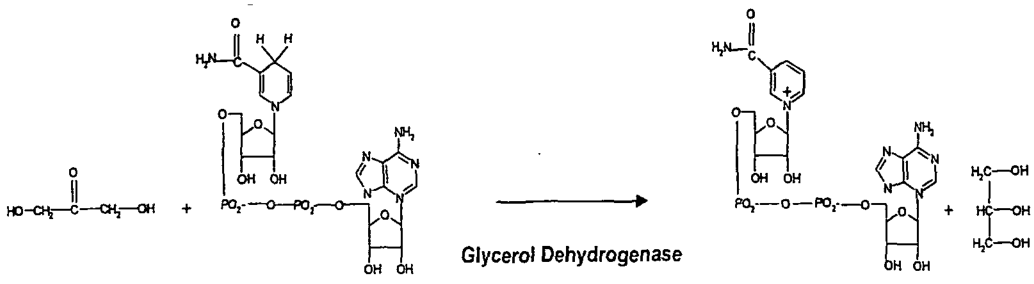


Dihydroxyaceton + NADH　　　　　　$NAD^+$ + Glycerol

$\lambda_{max.}$ = 365nm　　　　　　$\lambda_{max.}$ = 260nm

NADH = reduziertes Nicotinamid-adenin-dinucleotid.

Reagenzien:

Dihydroxyaceton (Merck), Glycylglycin (Merck), $(NH4)_2$ $SO_4$ (Merck), Sucrose (Merck), NADH-$Na_2$ (Merck), di-Natriumhydrogenphosphat-Dihydrat (Merck), Natriumdihydrogenphosphat-Dihydrat (Merck), Zinkchlorid (Merck), Glyceroldehydrogenase (Unitika Enzyms LTD).

DHA-Assay:

Die Bestimmung der DHA-Konzentration in einer Formulierung, basierend auf dem enzymatischen DHA-Assay, wie folgt beschrieben, basiert auf der folgenden Reaktion von Dihydroxyaceton:

$$DHA + NADH + H+ \rightarrow NAD+ + Glycerol$$

NADH zeigt einen Absorptionskoeffizienten von 365 nm. Das Enzym Glycerol-Dehydrogenase katalysiert die oben genannte Reaktion des Dihydroxyacetons mit NADH. Je mehr DHA vorhanden ist, desto mehr NADH wird in der Reaktion umgesetzt und die NADH Absorption bei 365 nm nimmt ab.

A. **Probenpuffer-Lösung:** 1,06g Glycylglycin, 0,42g $(NH_4)_2$ $SO_4$ und 10,0g Sucrose werden in demineralisiertem Wasser gelöst und der pH-Wert wird mit NaOH-Lösung (10%) auf 9.0 eingestellt und die Probe auf ein Volumen von 100ml aufgefüllt.

B. **NADH - $Na_2$-Lösung:** 0.060 g NADH-$Na_2$ wird in 10.0 ml demineralisiertem Wasser gelöst.

C. **Puffer für die Glycerin Dehydrogenase-Lösung:**

a. 4.43 g Di-natriumhydrogenphosphat,
b. 3.90 g Natriumdihydrogenphosphat-Dihydrat,
c. 100.0 g Sucrose
d. 0.003 g Zinkchlorid.

Alle genannten Substanzen werden in 800 ml demineralisiertem Wasser gelöst und die Lösung mit 1 N Salzsäure oder Natronlauge auf einen pH-Wert von 7,0 eingestellt. Danach wird das Volumen der Probe mit demineralisiertem Wasser auf 1000m1 aufgefüllt.

D. **Glycerin-Dehydrogenase-Lösung:** 10 mg Glycerindehydrogenase in 1ml GlycerinDehydrogenasePuffer (Lösung C).

E. **DHA-Standards:** 0.200 g DHA (Art.-No. 1.10150 Merck) wird in 100 ml demineralisiertem Wasser gelöst. Diese Lösung wird 1:10 verdünnt. Der Standard wird zweifach, unabhängig von einander, hergestellt. Die Abweichung beider Standards vom therotischen DHA-Gehalt muss 100% +/- 5% betragen.

Probenzubereitung:

0.5 g - 1.0 g der Testemulsion wird in einen kalibrierten 100 ml Kolben eingewogen. 60 ml heisses (70°C)

demineralisiertes Wasser wird eingefüllt und es wird gerührt, wobei die Temperatur bei 60°C für 15 Minuten konstant bleibt. Wenn die Lösung abgekühlt ist, wird bis zur Kalibrierungsmarke aufgefüllt. Die erhaltenen Lösungen werden über einen Membranfilter bis zur klaren Lösung filtriert. Von dieser Lösung wird ein Volumen von 0.1 ml zur DHA-Bestimmung verwendet.

Messbedingungen:

Cary 300 UV-Vis Spektrophotometer (Varian) (Wellenlänge = 365 nm, d = 1cm). Als Referenz wird demineralisiertes Wasser verwendet.

Pipettenprozedur:

| | Leerwert Lösung | Probe / Standard-Lösung |
|---|---|---|
| Probenpuffer | 1.00 ml | 1.00 ml |
| NADH-$Na_2$ | 0.10 ml | 0.10 ml |
| Demineralisiertes Wasser | 2.00 ml | 1.90 ml |
| Probe oder Standard | ----- | 0.10 mol |

Die Absorption dieser Lösungen ist E1 (Messung 5 Minuten nach Mischung)

Die Lösungen werden wie oben beschrieben gemischt und vermessen bevor das Enzym hinzugegeben wird. Für jede Probe ist die Extinktion vor Enzymzugabe E1.

Nachfolgende Zugabe der Glycerin-Dehydrogenase-Lösung jeweils 0.01 ml.

Die Absorption dieser Lösungen ist E2 (Messung nach 15 Minuten nach Zugabe der Enzymlösung).

$$\Delta E = E1 - E2$$

V = Totalvolumen : 3.11 ml
v = Probenvolumen : 0.1 ml
$\varepsilon = \varepsilon$ (NADH $Na_2$) = 3.42 $l{*}mmol^{-1}{*}cm^{-1}$
MW = Molmasse (DHA) : 90.08 g / mol
EW = Probenlösungkonzentration (g/L)

$$\text{Gehalt von DHA [Gew.-\% in Testformulierung]} = \frac{\Delta E * V * MW * 100}{\varepsilon * d * v * 1000 * EW\ (g/L)}$$

Mit dieser beschriebenen enzymbasierten Methode wird der Abbau in den Testformulierungen bestimmt, wie in den folgenden Beispielen angegeben: Die Abkürzung RT entspricht dem Begriff Raumtemperatur.

Ergebnisse:

Tabelle 1: Der DHA-Gehalt beträgt immer 5 Gewichtsprozent. Die folgende Tabelle gibt die Abnahme des DHA-Gehalts nach Lagerung in Dunkelheit bei Raumtemperatur in Gramm an:

| Typanorganischer Filter/DHA | Gehalt anorganischer UV-Filter | Gehalt Cetylphosphat | Abnahme des DHA-Gehalts nach ca. 1 Monat | Abnahme des DHA-Gehalts nach 2 Monaten | Abnahme des DHA-Gehalts nach 3 Monaten |
|---|---|---|---|---|---|
| **DHA (pur)** | 0g | 0% | 0,0g | 0,2g | 0,1g |

(fortgesetzt)

| Typ anorganischer Filter/DHA | Gehalt anorganischer UV-Filter | Gehalt Cetylphosphat | Abnahme des DHA-Gehalts nach ca. 1 Monat | Abnahme des DHA-Gehalts nach 2 Monaten | Abnahme des DHA-Gehalts nach 3 Monaten |
|---|---|---|---|---|---|
| DHA (pur), ($TiO_2$ 80%, $SiO_2$ 20%) | 5g | 0% | 1,1g | 1,7g | 1,8g |
| DHA (pur), ($TiO_2$ 80%, $SiO_2$ 20%) | 5g | 0,3% | 0,2g | 0,7g | 0,5g |
| DHA (pur) ($TiO_2$ 87%, $Al_2O_3$ 3%, Triethoxycaprylylsilan 9%) | 5g | 0% | 0,7g | 1,5g | 1,8g |
| DHA (pur) ($TiO_2$ 87%, $Al_2O_3$ 3%, Triethoxycaprylylsilan 9%) | 5g | 0,3% | 0,3g | 0,6g | 1,1g |

Tabelle 2: Der DHA-Gehalt beträgt immer 5 Gewichtsprozent. Die folgende Tabelle gibt die Abnahme des DHA-Gehalts nach Lagerung in Dunkelheit bei 40°C in Gramm an:

| Typ anorganischer Filter/DHA | Gehalt anorganischer UV-Filter | Gehalt Cetylphosphat | Abnahme des DHA-Gehalts nach ca. 1 Monat | Abnahme des DHA-Gehalts nach 2 Monaten | Abnahme des DHA-Gehalts nach 3 Monaten |
|---|---|---|---|---|---|
| DHA (pur) | 0g | 0% | 0,1g | 0,9g | 0,9g |
| DHA (pur), ($TiO_2$ 80%, $SiO_2$ 20%) | 5g | 0% | 3,9g | 4,4g | 4,4g |
| DHA (pur), ($TiO_2$ 80%, $SiO_2$ 20%) | 5g | 0,3% | 1,4g | 1,2g | 2,0g |
| DHA (pur) ($TiO_2$ 87%, $Al_2O_3$ 3%, Triethoxycaprylylsilan 9%) | 5g | 0% | 3,6g | 4,7g | 4,8g |
| DHA (pur) ($TiO_2$ 87%, $Al_2O_3$ 3%, Triethoxycaprylylsilan 9%) | 5g | 0,3% | 2,6g | 4,2g | 4,3g |

Die Anwesenheit von Cetylphosphat in der Zubereitung bewirkt deutlich eine geringere Abnahme des Gehalts an DHA in der Zubereitung im Vergleich zur Zubereitung ohne Cetylphosphat und dem angezeigten anorganischen UV-Filter.

Beispiel 1B: Es wurde der Einfluss des Gehalts an Cetylphosphat in der Testformulierung nach Beispiel 1A ermittelt.

Die Tabelle 3 fasst die Ergebnisse wie folgt zusammen. Es wird die Abnahme des DHA-Gehalts nach Lagerung in Dunkelheit bei Raumtempertur angegeben in Gramm:

| Typ anorganischer Filter/DHA | Gehalt anorganischer UV-Filter | Gehalt Cetylphosphat | Abnahme des DHA-Gehalts nach 3 Monaten |
|---|---|---|---|
| DHA (pur) | 0g | 0% | 0,1g |

(fortgesetzt)

| Typ anorganischer Filter/DHA | Gehalt anorganis cher UV-Filter | Gehalt Cetylphosphat | Abnahme des DHA-Gehalts nach 3 Monaten |
|---|---|---|---|
| DHA (pur), ($TiO_2$ 80%, $SiO_2$ 20%) | 5g | 0% | 2,1g |
| DHA (pur), ($TiO_2$ 80%, $SiO_2$ 20%) | 5g | 0,15% | 1,2g |
| DHA (pur), ($TiO_2$ 80%, $SiO_2$ 20%) | 5g | 0,3% | 0,8g |
| DHA (pur), ($TiO_2$ 80%, $SiO_2$ 20%) | 5g | 0,75% | 0,5g |
| DHA (pur), ($TiO_2$ 80%, $SiO_2$ 20%) | 5g | 1,5% | 0,3g |

Die Tabelle 4 fasst die Ergebnisse wie folgt zusammen. Es wird die Abnahme des DHA-Gehalts nach Lagerung in Dunkelheit bei 40°C angegeben in Gramm:

| Typ anorganischer Filter/DHA | Gehalt anorganis cher UV-Filter | Gehalt Cetylphosphat | Abnahme des DHA-Gehalts nach 3 Monaten |
|---|---|---|---|
| DHA (pur) | 0g | 0% | 0,9g |
| DHA (pur), ($TiO_2$ 80%, $SiO_2$ 20%) | 5g | 0% | 4,1g |
| DHA (pur), ($TiO_2$ 80%, $SiO_2$ 20%) | 5g | 0,15% | 3,4g |
| DHA (pur), ($TiO_2$ 80%, $SiO_2$ 20%) | 5g | 0,3% | 1,8g |
| DHA (pur), ($TiO_2$ 80%, $SiO_2$ 20%) | 5g | 0,75% | Nicht stabil |
| DHA (pur), ($TiO_2$ 80%, $SiO_2$ 20%) | 5g | 1,5% | Nicht stabil |

Beispiel 2: In den Untersuchungen wurde die nachfolgend beschriebene Hydrogel-Testformulierung verwendet. Die Testformulierung wurde im 200g-Maßstab angesetzt:

| **Handelsname** | | **0-1-00** | **0-1-01** | **0-1-03** | **0-1-04** |
|---|---|---|---|---|---|
| | | **Einwaage** | **Einwaage** | **Einwaage** | **Einwaage** |
| **A** | | **g** | **g** | **g** | **g** |
| Placebo ohne $TiO_2$ | | **0,00** | | | |
| **$TiO_2$ 80%, $SiO_2$ 20%** | **2,00** | | **4,00** | | **4,00** |
| | | | | | |
| Cetylphosphat | **0,12** | | | **0,24** | **0,24** |
| Wasser, demineralisiert | **11,98** | **32,20** | **28,20** | **31,96** | **27,96** |
| **B** | | | | | |
| Dihydroxyaceton | **2,00** | **4,00** | *4,00* | *4,00* | *4,00* |
| Natriumdisulfit | **0,20** | **0,40** | *0,40* | *0,40* | *0,40* |
| Transcutol CG | **1,00** | **2,00** | *2,00* | *2,00* | *2,00* |

(fortgesetzt)

| | | | | | |
|---|---|---|---|---|---|
| **B** | | | | | |
| 1,2-Propandiol | **2,00** | **4,00** | *4,00* | *4,00* | *4,00* |
| Karion F flüssig | **2,00** | **4,00** | *4,00* | *4,00* | *4,00* |
| Methyl-4-hydroxybenzoat | **0,20** | **0,40** | *0,40* | *0,40* | *0,40* |
| Wasser, demineralisiert | **15,00** | **30,00** | *30,00* | *30,00* | *30,00* |
| **C** | | | | | |
| Natrosol 250 HHR | **1,50** | **3,00** | *3,00* | *3,00* | *3,00* |
| Wasser | **60,00** | **120,00** | *120,00* | *120,00* | *120,00* |
| | **100,00** | **200,00** | **200,00** | **200,00** | **200,00** |

**Herstellung:**

Herstellung über Masterbatch:

Für **Phase C** wird das Natrosol langsam in den Wirbel des kräftig gerührten Wassers gegeben. Die Zugabe soll langsam und gleichmäßig erfolgen, so daß die Partikel sich im Wasser ohne Klumpenbildung verteilen können, aber nicht so langsam, daß die Lösung sich stark verdickt, bevor alles zugegeben ist.

Für **Phase B** wird Dihydroxyaceton in Wasser gelöst, die restlichen Rohstoffe werden unter Rühren zugegeben. Diese Lösung wird in **Phase C** eingerührt (400 rpm).

**Phase A** wird jeweils in einem 400ml Becherglas vorgelegt und 167,8 g der **Mischphase B&C** hinzugegeben. Diese Mischung wird 1 min mit den ESGE Zauberstab auf Stufe II homogenisiert.

Die Testformulierung enthält immer 2 Gewichtsprozent DHA und 2 Gewichtsprozent Metalloxid und/oder Metallhydroxid.

Die DHA-Bestimmung erfolgt nach der Methode, wie unter Beispiel 1A angegeben.

Ergebnisse:

Die Tabelle 5 fasst die Ergebnisse wie folgt zusammen. Es wird die Abnahme des DHA-Gehalts nach Lagerung in Dunkelheit bei Raumtemperatur angegeben (in Gramm):

| Typ anorganischer Filter/DHA | Gehalt anorganis cherUV-Filter | Gehalt Cetylphosphat | Abnahme des DHA-Gehalts nach 1 Woche. | Abnahme des DHA-Gehalts nach 1 Monat |
|---|---|---|---|---|
| **DHA (pur)** | 0% | 0% | 0,1g | 0,1g |
| **DHA (pur), ($TiO_2$ 80%, $SiO_2$ 20%)** | 2q | 0% | 1,3g | 1,6g |
| **DHA (pur), ($TiO_2$ 80%, $SiO_2$ 20%)** | 2g | 0,12% | 0,7g | 1,3g |

Es hat sich herausgestellt, dass die Testformulierung bei Lagerung bei 40°C nicht stabil ist, wenn Titandioxid ($TiO_2$ 80%, $SiO_2$ 20%) anwesend ist.

Beispiel 3:

Bei den Untersuchungen des Beispiels 3 wurden Formulierungen nach Beispiel 1A hergestellt, wobei die organische Phosphorverbindung wie angezeigt variiert wurde. Die Testformulierung wurde im 50g-Maßstab angesetzt. Die Ergebnisse werden in Tabelle 6 zusammengefasst. Es wird die Abnahme des DHA-Gehalts nach Lagerung in Dunkelheit bei 40°C nach 14 Tagen angegeben.

Tabelle 6:

| Typ Phosphorverbindu ng | Gehalt ($TiO_2$ 80%, $SiO_2$ 20%) | Gehalt Phosphorverbindung | Abnahme des DHA-Gehalts in % bezogen auf den Restgehalt an DHA in Placebo |
|---|---|---|---|
| **Ohne Phosphorverbindu ng** | 2,5g | | 15% |
| **Cetyl phosphate** | 2,5g | 0,38g | 0% |
| **Monostearylphosp horic acid eser/Distearyl phosphoric acid ester** | 2,5g | 0,38g | 0% |
| **Monostearylphosp horic acid eser/Distearyl phosphoric acid este** | 2,5g | 0,75g | 2% |

Bei Lagerung von 3 Monaten bei 40°C der Testformulierung mit Cetylphosphat wurde eine Abnahme des DHA-Gehalts von 22% bezogen auf den Restgehalt an DHA in Placebo beobachtet.

Der Begriff Placebo bedeutet, dass die gleiche Testformulierung enthaltend alle Zubereitungsbestandteile (einschließlich DHA) ausser dem Metalloxid und/oder Metallhydroxid und der organischen Phosphorverbindung untersucht wird. Auch bei der Placebo-Formulierung wird ein Abbau von DHA nach Lagerung von 3 Monaten bei 40°C in Dunkelheit beobachtet. Dieser DHA-Gehalt wird als Grundlage für die Berechnung verwendet.

## Patentansprüche

1. Verfahren zur Herstellung einer Zubereitung in Form einer Öl-in-Wasser-Emulsion, **dadurch gekennzeichnet, dass** der Ölphase der Zubereitung mindestens eine organische Phosphorverbindung und anschließend ein Metalloxid und/oder Metallhydroxid beigemischt wird und bei maximal 40°C die mindestens eine wässrige Phase enthaltend Dihydroxyaceton zugegeben und homogenisiert wird, wobei das Metalloxid und/oder -hydroxid aus der Gruppe der Oxide bzw. Hydroxid von Silizium, Titan, Zink, Aluminium, Cer, Eisen, Yttrium oder Zirkonium oder Mischungen hieraus ausgewählt wird, die mit Metalloxiden bzw. -hydroxiden, organischen Säuren, ausgewählt aus der Gruppe Stearinsäure, Laurinsäure, Capronsäure oder Palmitinsäure, Polyolen, Polymeren oder silikonorganischen Verbindungen beschichtet sein können oder eine manganhaltige Beschichtung enthalten können, und wobei die mindestens eine organische Phosphorverbindung ausgewählt wird aus der Gruppe der Hydroxyalkyldiphosphonsäuren, Alkylphosphonsäuren, die durch mindestens eine COOH-Gruppe substituiert sein können, Aminoalkylenphosphonsäuren oder organischen Phosphorsäureestern oder deren Salze.

2. Verfahren zur Herstellung einer Zubereitung in Form eines Hydrogels, **dadurch gekennzeichnet, dass** eine wässrige Lösung enthaltend mindestens eine organische Phosphorverbindung und ein Metalloxid und/oder Metallhydroxid vorgelegt wird und eine Mischung aus einem Gelbildner und Dihydroxyaceton in Wasser zugegeben wird und homogenisiert wird, wobei das Metalloxid und/oder -hydroxid aus der Gruppe der Oxide bzw. Hydroxide von Silizium, Titan, Zink, Aluminium, Cer, Eisen, Yttrium oder Zirkonium oder Mischungen hieraus ausgewählt wird, die mit Metalloxiden bzw. -hydroxiden, organischen Säuren, ausgewählt aus der Gruppe Stearinsäure, Laurinsäure, Capronsäure oder Palmitinsäure, Polyolen, Polymeren oder silikonorganischen Verbindungen beschichtet sein können oder eine manganhaltige Beschichtung enthalten können, und wobei die mindestens eine organische Phosphorverbindung ausgewählt wird aus der Gruppe der Hydroxyalkyldiphosphonsäuren, Alkylphosphonsäuren, die durch mindestens eine COOH-Gruppe substituiert sein können, Aminoalkylenphosphonsäuren oder organischen Phosphorsäureestern oder deren Salze.

3. Verfahren nach Anspruch 1 oder 2, **dadurch gekennzeichnet, dass** die mindestens eine Phosphorverbindung ausgewählt wird aus Hydroxyalkyldiphosphonsäuren der Formel I

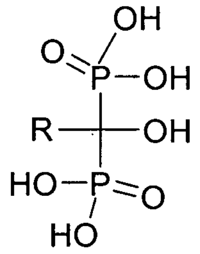

I

,

wobei R eine lineare oder verzweigte Alkylgruppe mit 1 bis 20 C-Atomen bedeutet oder deren Salze oder, dass die mindestens eine Phosphorverbindung ausgewählt wird aus Alkylphosphonsäuren mit einer linearen oder verzweigten Alkylgruppe mit 1 bis 20 C-Atomen, die gegebenenfalls mit mindestens einer COOH-Gruppe ersetzt sein können oder deren Salze oder,
dass die mindestens eine Phosphorverbindung ausgewählt wird aus Aminoalkylenphosphonsäuren der Formel II,

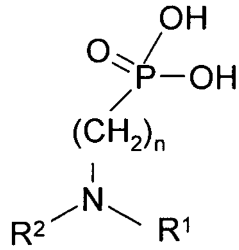

II

,

wobei n 1, 2, 3 oder 4 bedeutet, vorzugsweise 1 oder 2, und
$R^1$ und $R^2$ stehen jeweils unabhängig voneinander für $-(CH_2)_{n1}-P(O)(OH)_2$ oder $-(CH_2)_{n1}-N\{[(CH_2)_{n2}-P(O)(OH)_2][(CH_2)_{n3}-P(O)(OH)_2]\}$, wobei n1, n2 oder n3 jeweils unabhängig 1, 2, 3 oder 4 bedeuten oder deren Salze.

4. Verfahren nach Anspruch 1 oder 2, **dadurch gekennzeichnet, dass** die mindestens eine Phosphorverbindung ausgewählt wird aus Phosphorsäuremonoalkylestern mit einer linearen oder verzweigten Alkylgruppe mit 1 bis 20 C-Atomen, Phosphorsäuredialkylestern mit linearen oder verzweigten Alkylgruppen mit 1 bis 20 C-Atomen, wobei die Alkylgruppen jeweils unabhängig voneinander ausgewählt werden, Phosphorsäuretrialkylestern mit linearen oder verzweigten Alkylgruppen mit 1 bis 20 C-Atomen, wobei die Alkylgruppen jeweils unabhängig voneinander ausgewählt werden, Salze der Phosphorsäuremonoalkylester oder Phosphorsäuredialkylester oder Gemische dieser Phosphorsäureester, Phosphorsäure-Oxiestern, (3-Aminopropyl)-tocopheryl-phosphorsäureester oder Nucleotiden.

5. Verfahren nach einem oder mehreren der Ansprüche 1 bis 4, **dadurch gekennzeichnet, dass** das Metalloxid und/oder -hydroxid ausgewählt wird aus Titandioxid oder Titanhydroxid oder Mischungen hieraus, die mit Metalloxiden oder -hydroxiden von Silizium, Zirkonium, Mangan und/oder Aluminium, mit Stearinsäure, Laurinsäure, Capronsäure oder Palmitinsäure, mit Glycerin, Sorbitol, Propylenglycol, Pentandiol, Hexandiol, Ethylhexyl-Glycerin, Pentaerythritol, Dipentaerythritol oder Trimethylolpropan oder mit Octyltrimethylsilan, Methicon, Dimethicon (= Triethoxy-caprylsilan), Trimethoxycaprylsilan, Dimethicon/Methicon Copolymer, Diphenyl Capryl Methicone, Polymethylmethacrylate dimethicone, Simethicon, Polyvinylpyrrolidon oder Polyethylen beschichtet sein können.

6. Verfahren nach einem oder mehreren der Ansprüche 1 bis 5, **dadurch gekennzeichnet, dass** der Anteil an der mindestens einen organischen Phosphorverbindung in Gewichtsprozent bezogen auf den Anteil des Metalloxids oder Metallhydroxids in der Zubereitung im Bereich von 3 Gewichtsprozent bis 25 Gewichtsprozent liegt.

7. Verfahren nach einem oder mehreren der Ansprüche 1 bis 6, **dadurch gekennzeichnet, dass** der Anteil an Dihydroxyacdeton im Bereich von 1 Gewichtsprozent bis 12 Gewichtsprozent liegt, bezogen auf die Zubereitung.

8. Verfahren nach einem oder mehreren der Ansprüche 1 bis 7, **dadurch gekennzeichnet, dass** der Anteil an dem mindestens einen Metalloxid und/oder Metallhydroxid im Bereich von 0,5 Gewichtsprozent bis 25 Gewichtsprozent

liegt, bezogen auf die Zubereitung.

## Claims

1. Process for the preparation of a preparation in the form of an oil-in-water emulsion, **characterised in that** at least one organic phosphorus compound and subsequently a metal oxide and/or metal hydroxide are admixed with the oil phase of the preparation, and the at least one aqueous phase containing dihydroxyacetone is added at a maximum of 40°C and homogenised, where the metal oxide and/or hydroxide is selected from the group of the oxides or hydroxides respectively of silicon, titanium, zinc, aluminium, cerium, iron, yttrium or zirconium or mixtures thereof, which may be coated with metal oxides or hydroxides, organic acids selected from the group stearic acid, lauric acid, caproic acid or palmitic acid, polyols, polymers or organosilicone compounds or may contain a manganese-containing coating, and where the at least one organic phosphorus compound is selected from the group of the hydroxyalkyldiphosphonic acids, alkylphosphonic acids, which may be substituted by at least one COOH group, aminoalkylenephosphonic acids or organic phosphoric acid esters or salts thereof.

2. Process for the preparation of a preparation in the form of a hydrogel, **characterised in that** an aqueous solution containing at least one organic phosphorus compound and a metal oxide and/or metal hydroxide is initially introduced and a mixture of a gel former and dihydroxyacetone in water is added and homogenised, where the metal oxide and/or hydroxide is selected from the group of the oxides or hydroxides respectively of silicon, titanium, zinc, aluminium, cerium, iron, yttrium or zirconium or mixtures thereof, which may be coated with metal oxides or hydroxides, organic acids selected from the group stearic acid, lauric acid, caproic acid or palmitic acid, polyols, polymers or organosilicone compounds or may contain a manganese-containing coating, and where the at least one organic phosphorus compound is selected from the group of the hydroxyalkyldiphosphonic acids, alkylphosphonic acids, which may be substituted by at least one COOH group, aminoalkylenephosphonic acids or organic phosphoric acid esters or salts thereof.

3. Process according to Claim 1 or 2, **characterised in that** the at least one phosphorus compound is selected from hydroxyalkyldiphosphonic acids of the formula I

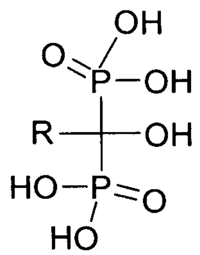

I

,

where R denotes a linear or branched alkyl group having 1 to 20 C atoms or salts thereof or
**in that** the at least one phosphorus compound is selected from alkylphosphonic acids containing a linear or branched alkyl group having 1 to 20 C atoms, which may optionally be replaced by at least one COOH group, or salts thereof, or
**in that** the at least one phosphorus compound is selected from aminoalkylenephosphonic acids of the formula II,

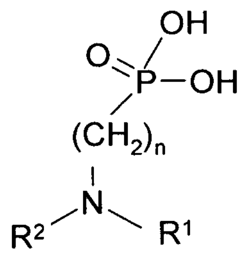

II

,

where n denotes 1, 2, 3 or 4, preferably 1 or 2, and
$R^1$ and $R^2$ each stand, independently of one another, for $-(CH_2)_{n1}-P(O)(OH)_2$ or

$-(CH_2)_{n1}-N\{[(CH_2)_{n2}-P(O)(OH)_2][(CH_2)_{n3}-P(O)(OH)_2]\}$, where n1, n2 or n3 each independently denote 1, 2, 3 or 4, or salts thereof.

4. Process according to Claim 1 or 2, **characterised in that** the at least one phosphorus compound is selected from monoalkyl phosphates containing a linear or branched alkyl group having 1 to 20 C atoms, dialkyl phosphates containing linear or branched alkyl groups having 1 to 20 C atoms, where the alkyl groups are each selected independently of one another, trialkyl phosphates containing linear or branched alkyl groups having 1 to 20 C atoms, where the alkyl groups are each selected independently of one another, salts of the monoalkyl phosphates or dialkyl phosphates or mixtures of these phosphates, phosphoric acid oxyesters, 3-aminopropyl tocopheryl phosphate or nucleotides.

5. Process according to one or more of Claims 1 to 4, **characterised in that** the metal oxide and/or hydroxide is selected from titanium dioxide or titanium hydroxide or mixtures thereof, which may be coated with metal oxides or hydroxides of silicon, zirconium, manganese and/or aluminium, with stearic acid, lauric acid, caproic acid or palmitic acid, with glycerol, sorbitol, propylene glycol, pentanediol, hexanediol, ethylhexyl-glycerol, pentaerythritol, dipentaerythritol or trimethylolpropane or with octyltrimethylsilane, methicone, dimethicone (= triethoxycaprylsilane), trimethoxycaprylsilane, dimethicone/methicone copolymer, Diphenyl Capryl Methicone, polymethyl methacrylate dimethicone, simethicone, polyvinylpyrrolidone or polyethylene.

6. Process according to one or more of Claims 1 to 5, **characterised in that** the proportion of the at least one organic phosphorus compound in per cent by weight, based on the proportion of the metal oxide or metal hydroxide in the preparation is in the range from 3 per cent by weight to 25 per cent by weight.

7. Process according to one or more of Claims 1 to 6, **characterised in that** the proportion of dihydroxyacetone is in the range from 1 per cent by weight to 12 per cent by weight, based on the preparation.

8. Process according to one or more of Claims 1 to 7, **characterised in that** the proportion of the at least one metal oxide and/or metal hydroxide is in the range from 0.5 per cent by weight to 25 per cent by weight, based on the preparation.

## Revendications

1. Procédé de préparation d’une préparation sous la forme d’une émulsion huile-dans-eau, **caractérisé en ce qu’**au moins un composé de phosphore organique puis un oxyde métallique et/ou un hydroxyde métallique sont mélangés avec la phase huileuse de la préparation, et la au moins une phase aqueuse contenant de la dihydroxyacétone est ajoutée à un maximum de 40°C et homogénéisée, où l’oxyde et/ou hydroxyde métallique est choisi dans le groupe constitué par les oxydes ou hydroxydes respectivement de silicium, titane, zinc, aluminium, cérium, fer, yttrium ou zirconium ou des mélanges de ceux-ci, pouvant être revêtu par des oxydes ou hydroxydes métalliques, des acides organiques choisis dans le groupe constitué par l’acide stéarique, l’acide laurique, l’acide caproïque ou l’acide palmitique, les polyols, les polymères ou des composés d’organo-silicone ou pouvant contenir un revêtement à base de manganèse, et où le au moins un composé de phosphore organique est choisi dans le groupe constitué par les acides hydroxyalkyldiphosphoniques, les acides alkylphosphoniques, pouvant être substitués par au moins un groupement COOH, les acides aminoalkylènephosphoniques ou les esters d’acide phosphorique organiques ou des sels de ceux-ci.

2. Procédé de préparation d’une préparation sous la forme d’un hydrogel, **caractérisé en ce qu’**une solution aqueuse contenant au moins un composé de phosphore organique et un oxyde métallique et/ou un hydroxyde métallique est initialement introduite et un mélange d’un agent formateur de gel et de dihydroxyacétone dans l’eau est ajouté et homogénéisé, où l’oxyde et/ou hydroxyde métallique est choisi dans le groupe constitué par les oxydes ou hydroxydes respectivement de silicium, titane, zinc, aluminium, cérium, fer, yttrium ou zirconium ou des mélanges de ceux-ci, pouvant être revêtu par des oxydes ou hydroxydes métalliques, des acides organiques choisis dans le groupe constitué par l’acide stéarique, l’acide laurique, l’acide caproïque ou l’acide palmitique, les polyols, les polymères ou des composés d’organo-silicone ou pouvant contenir un revêtement à base de manganèse, et où le au moins un composé de phosphore organique est choisi dans le groupe constitué par les acides hydroxyalkyldiphosphoniques, les acides alkylphosphoniques, pouvant être substitués par au moins un groupement COOH, les acides aminoalkylènephosphoniques ou les esters d’acide phosphorique organiques ou des sels de ceux-ci.

3. Procédé selon la revendication 1 ou 2, **caractérisé en ce que** le au moins un composé de phosphore est choisi parmi les acides hydroxyalkyldiphosphoniques de formule I

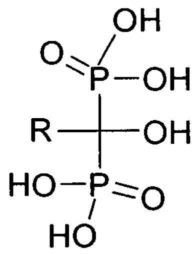

I

,

où R désigne un groupement alkyle linéaire ou ramifié ayant de 1 à 20 atomes de C ou des sels de ceux-ci ou **en ce que** le au moins un composé de phosphore est choisi parmi les acides alkylphosphoniques contenant un groupement alkyle linéaire ou ramifié ayant de 1 à 20 atomes de C, pouvant éventuellement être remplacé par au moins un groupement COOH, ou des sels de ceux-ci, ou
**en ce que** le au moins un composé de phosphore est choisi parmi les acides aminoalkylènephosphoniques de formule II,

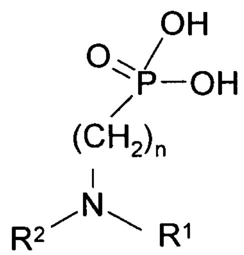

II

,

où n désigne 1, 2, 3 ou 4, préférablement 1 ou 2, et
$R^1$ et $R^2$ représentent chacun, indépendamment l'un de l'autre, $-(CH_2)_{n1}-P(O)(OH)_2$ ou $-(CH_2)_{n1}-N\{[(CH_2)_{n2}-P(O)(OH)_2][(CH_2)_{n3}-P(O)(OH)_2]\}$, où n1, n2 ou n3 désignent chacun indépendamment 1, 2, 3 ou 4, ou des sels de ceux-ci.

4. Procédé selon la revendication 1 ou 2, **caractérisé en ce que** le au moins un composé de phosphore est choisi parmi les phosphates de monoalkyle contenant un groupement alkyle linéaire ou ramifié ayant de 1 à 20 atomes de C, les phosphates de dialkyle contenant des groupements alkyle linéaires ou ramifiés ayant de 1 à 20 atomes de C, où les groupements alkyle sont choisis chacun indépendamment les uns des autres, les phosphates de trialkyle contenant des groupements alkyle linéaires ou ramifiés ayant de 1 à 20 atomes de C, où les groupements alkyle sont choisis chacun indépendamment les uns des autres, les sels des phosphates de monoalkyle ou des phosphates de dialkyle ou des mélanges de ces phosphates, les oxyesters d'acide phosphorique, le phosphate de 3-aminopropyle et de tocophéryle ou les nucléotides.

5. Procédé selon l'une ou plusieurs parmi les revendications 1 à 4, **caractérisé en ce que** l'oxyde et/ou hydroxyde métallique est choisi parmi le dioxyde de titane ou l'hydroxyde de titane ou des mélanges de ceux-ci, pouvant être revêtu par des oxydes ou hydroxydes métalliques de silicium, zirconium, manganèse et/ou aluminium, par de l'acide stéarique, de l'acide laurique, de l'acide caproïque ou de l'acide palmitique, par du glycérol, du sorbitol, du propylène glycol, du pentanediol, de l'hexanediol, de l'éthylhexylglycérol, du pentaérythritol, du dipentaérythritol ou du triméthylolpropane ou par de l'octyltriméthylsilane, de la méthicone, de la diméthicone (= triéthoxycaprylsilane), du triméthoxycaprylsilane, un copolymère de diméthicone/méthicone, de la diphényl capryl méthicone, du méthacrylate de polyméthyle diméthicone, de la siméthicone, de la polyvinylpyrrolidone ou du polyéthylène.

6. Procédé selon l'une ou plusieurs parmi les revendications 1 à 5, **caractérisé en ce que** la proportion du au moins un composé de phosphore organique en pourcentage en poids, sur la base de la proportion de l'oxyde métallique ou de l'hydroxyde métallique dans la préparation se trouve dans la plage allant de 3 pour cent en poids à 25 pour cent en poids.

7. Procédé selon l'une ou plusieurs parmi les revendications 1 à 6, **caractérisé en ce que** la proportion de dihydroxyacétone se trouve dans la plage allant de 1 pour cent en poids à 12 pour cent en poids, sur la base de la préparation.

8. Procédé selon l'une ou plusieurs parmi les revendications 1 à 7, **caractérisé en ce que** la proportion du au moins un oxyde métallique et/ou hydroxyde métallique se trouve dans la plage allant de 0,5 pour cent en poids à 25 pour cent en poids, sur la base de la préparation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 9217159 A2
- EP 576188 A1
- DE 102007013368
- FR 2326405 A
- FR 2440933 A
- EP 0114607 A
- WO 9304665 A
- EP 0487404 A
- US 6242099 B1
- WO 0009652 A
- WO 0072806 A
- WO 0071084 A
- DE 4116123 A

### In der Beschreibung aufgeführte Nicht-Patentliteratur

- Colour Index Nummern (CIN) sind dem Rowe Colour Index. Society of Dyers and Colourists, 1971